# EUROPÄISCHE PATENTSCHRIFT

(11) **EP 2 752 421 B1**
(45) Veröffentlichungstag und Bekanntmachung des Hinweises auf die Patenterteilung: **07.06.2023**
(21) Anmeldenummer: 14162852.9
(22) Anmeldetag: 08.02.2011
(51) Int. Cl.: C07H 9/04, B29C 64/135, B33Y 70/00

(54) **VERFAHREN ZUM HERSTELLEN VON (METH)ACRYLAT-SUBSTITUIERTEN MONOSACCHARIDEN, ZUCKERALKOHOLEN, MONO-HYDROXYSÄUREN UND DEREN LACTONE UND LACTIDE, MIT DIESEM VERFAHREN HERGESTELLTE, TOXISCH UNBEDENKLICHE PRODUKTE SOWIE DEREN VERWENDUNG ZUM ERZEUGEN VON FORMKÖRPERN ODER STRUKTURIERTEN OBERFLÄCHEN**
METHOD FOR THE PRODUCTION OF (METH) ACRYLATE SUBSTITUTED MONOSACCHARIDES, SUGAR ALCOHOLS, MONO-HYDROXY ACIDS AND THEIR LACTONES AND LACTIDES WITH THIS METHOD, PRODUCTS WITH ACCEPTABLE LEVEL OF TOXICITY AND THEIR USE FOR PRODUCING SHAPED BODIES OR STRUCTURED SURFACES
PROCÉDÉ DE FABRICATION DE MONOSACCHARIDES (MÉTH)ACRYLATE-SUBSTITUÉS, D'ALCOOLS DE SUCRE, ET D'ACIDES MONO-HYDROXYLÉS ET DE LEURS LACTONES ET LACTIDES, PRODUITS INOFFENSIFS SUR LE PLAN TOXIQUE FABRIQUÉS SELON CE PROCÉDÉ ET LEUR UTILISATION POUR LA GÉNÉRATION DE CORPS DE MOULES OU DE SURFACES STRUCTURÉES

(30) Priorität: 12.02.2010 EP 10153550
(43) Veröffentlichungstag der Anmeldung: 09.07.2014
(62) Teilanmeldung aus: 11702070.1
(73) Patentinhaber: Fraunhofer-Gesellschaft zur Förderung der angewandten Forschung e.V., 80686 München (DE)
(72) Erfinder: Houbertz-Krauss, Ruth, 97074 Würzburg (DE); Beyer, Matthias, 76327 Pfinztal (DE); Probst, Jörn, 97273 Kürnach (DE); Stichel, Thomas, 97070 Wuerzburg (DE)
(74) Vertreter: Strehl Schübel-Hopf & Partner

(56) Entgegenhaltungen:
- WO-A1-2006/040470
- I. R. SALAGEAN ET AL: "Copolymerization of 3-O-Acryloyl-1,2:5,6-di-O-Isopropylidene-a lpha-D-Glucofuranose and Buthyl Acrylate at Different Molar Ratios", CHEM. BULL. "POLITEHNICA" UNIV. TIMISOARA (ROMANIA), Bd. 53, Nr. 1-2, 2008, Seiten 69-72, XP002590370,
- Morrison R. T. und Boyd R. N.: "LEHRBUCH DER ORGANISCHEN CHEMIE", 1974, verlag chemie, Weinheim, XP002632097, * pages732, 733, 734, 741,742 * * Absätze [20.15], [20.20] *

## Beschreibung

Die Erfindung betrifft ein Verfahren zum Herstellen einer Verbindung, die eingesetzt werden kann in Ausgangsmaterialien für die Erzeugung biologisch verträglicher, beliebig geformter Körper mit dreidimensionalen Strukturen, die entweder freitragend und/oder durch einen Träger gehalten sein können, durch Verfestigen einer polymerisierbaren Flüssigkeit.

Die Ausgangsmaterialien rufen keine oder nur tolerabel geringe toxische Reaktionen seitens der physiologischen Umgebung hervor.

Die Körper sind biologisch verträglich. Unter diesem Ausdruck soll verstanden werden, dass die Körper je nach Anwendungsgebiet in einem ersten Fall biodegradierbar, d.h. unter physiologischen Bedingungen durch im menschlichen oder tierischen Körper vorhandenen Enzyme oder sonstige Bestandteile oder durch die chemische Umgebung, in der sie sich befinden, zumindest teilweise zerlegbar sein sollen. Unter "zerlegbar" soll dabei verstanden werden, dass die physiologische Umgebung die Spaltung von chemischen - anorganischen, organischen oder anorganisch-organischen wie Kohlenstoff-Metall - Bindungen bewirkt, wodurch der Zusammenhalt des Körpers geschwächt wird oder Poren gebildet werden, bis der Körper im Extremfall in kleinere Bestandteile aufgelöst ist, die innerhalb des Körpers abtransportiert und letztendlich ausgeschieden oder weiter metabolisiert werden. Neben den genannten enzymatischen Vorgängen ist dabei vor allem auf das Auftreten einer unspezifischen Hydrolyse zu verweisen. Wird der biokompatible Körper ganz oder teilweise ohne nachhaltige Entzündungsreaktionen metabolisiert, soll er als bioresorbierbar gelten. Die Biodegradierbarkeit und ggf. Bioresorbierbarkeit der Körper wird z.B. dann gewünscht, wenn sie als Knochenimplantate eingesetzt werden sollen, deren Volumen im Lauf der Zeit durch körpereigenen Knochen ersetzt werden sollen. Ähnliches gilt für poröse Körper, die *in vitro* mit Zellen vorbesiedelt wurden und nach der Implantierung durch einen durchgehenden Zellverbund ersetzt werden sollen, z.B. einem Hautstück.

Jedoch ist nicht bei allen Implantaten eine teilweise oder vollständige Biodegradierbarkeit innerhalb eines überschaubaren Zeitraums wünschenswert. Knorpelgewebe beispielsweise bildet sich sehr langsam oder überhaupt nicht mehr vollständig nach, so dass Knorpel-Implantate zumindest sehr lange, wenn nicht für immer, wenigstens in Form eines Rest-Stützgerüsts im Körper, z.B. im Ohr, verbleiben sollten.

Die Erfindung betrifft die Herstellung von toxisch unbedenklichen Acrylsäure- und Methacrylsäurederivaten von monomeren und oligomeren Zuckern sowie Kohlehydraten. Mit "toxisch" werden im Rahmen der vorliegenden Erfindung Stoffe bezeichnet, die in physiologischer Umgebung unerwünschte Nebenwirkungen, insbesondere eine detektierbare, meist starke immunologische Abwehrreaktion hervorrufen.

Die genannte Aufgabe wird gelöst durch das in den angehängten Ansprüchen definierte Verfahren zum Herstellen einer Verbindung.

Die mit dem erfindungsgemäßen Verfahren hergestellte Verbindung eignet sich zum Erzeugen von dreidimensionalen selbsttragenden und/oder substratgestützten Formkörpern oder von dreidimensionale Oberflächenstrukturen durch ortsselektives Verfestigen eines flüssigen bis pastösen, organischen oder organisch modifizierten Materials innerhalb eines Bades aus diesem Material mit Hilfe von Zwei- oder Mehr-Photonen-Polymerisation, wobei das Material einen oder mehrere Bestandteile aufweist, ausgewählt unter Verbindungen mit einem organischen, über 2-Photonen- oder Mehrphotonen-Polymerisation polymerisierbaren Rest und/oder Verbindungen, die mindestens einen biokompatiblen, biodegradierbaren oder bioresorbierbaren Rest besitzen, mit der Maßgabe, dass in dem Material sowohl ein organischer, über 2-Photonen- oder Mehrphotonen-Polymerisation polymerisierbarer Rest als auch eine biokompatible, biodegradierbare oder bioresorbierbare Gruppe enthalten sein müssen. Als "dreidimensionale Oberflächenstruktur" ist eine aufgebrachte Schicht auf einem beliebig geformten, bereits bestehenden Körper zu verstehen. Das bedeutet, dass diese Schicht entweder bei im Wesentlichen gleich bleibender oder bei schwankender Dicke eine Oberflächenstrukturierung und/oder eine der Gestalt des bereits bestehenden Körpers folgende Kontur aufweist.

Die Technologie hinsichtlich des Materials und des Verfahrens zeichnet sich dadurch aus, dass
(a) beliebige dreidimensionale Trägerstrukturen direkt und schnell in einem Material hergestellt werden können,
(b) eine variable Porenstruktur vom 100 nm bis in den mm-Bereich eingestellt werden kann,
(c) eine wahlweise interkonnektierende Porenstruktur erhalten werden kann,
(d) eine großflächige Strukturierung bis in den cm-Maßstab mit hoher Präzision und sehr hoher Wiederholgenauigkeit durchgeführt werden kann,
(e) auf Sinterprozesse, wie sie in anderen Verfahren (s. a. Stand der Technik) angewendet werden müssen, verzichtet werden kann,
(f) beliebig viele extrem genaue Kopien der Strukturen erzeugt werden können,
(g) die Strukturen beliebig skalierbar sind,
(h) Patienten-individuelle Implantate hergestellt werden können, wobei die geometrischen Daten aus 3D-Scans (z. B. CT, Laser-Triangulation) direkt im Fertigungsprozess umgesetzt werden können,
(i) insbesondere auch sichtbares Licht sowie infra-rotes Licht durch Ultrakurzpulse, d. h. durch Anregung von Zwei- oder Mehr-Photonen-Prozesse, für die Herstellung der Strukturen verwendet werden kann,
(j) dioden-gepumpte Festkörperlaser oder Halbleiterlaser, Faserlaser, etc. verwendet werden können,
(k) in der Materialvorstufe auch biologische Komponenten integriert werden können.

Als biodegradierbare Trägermaterialien sind unter anderem die in der Einleitung genannten Materialien einsetzbar. Neben den dort genannten Polyestern kommen aber noch einige weitere Stoffklassen als biodegradierbare Strukturelemente in Frage. Diese sind insbesondere:
1. Kohlenhydrate (Mono-, Di- und Polysaccharide)
2. Aminosäuren, Peptide und Proteine, z.B. Kollagene oder Enzyme, sowie deren Lactam-Strukturen
3. Polyanhydride
4. Polyorthoester
5. Polyether
6. Polycarbonate
7. Polyamide
8. Monohydroxysäuren (insbesondere α- oder ω-Hydroxysäuren sowie deren Lactone und Lactide) sowie deren Oligomere oder Polymere

Der biokompatible, biodegradierbare oder bioresorbierbare Rest wird demnach je nach Bedarf vom Fachmann ausgewählt. Günstig sind Reste, die mindestens eine (und vorzugsweise 2 oder, stärker bevorzugt, mindestens 3) Gruppen aufweisen, die jeweils unabhängig voneinander ausgewählt sind unter -O-, -OC(O)O-, -C(O)NH-, -NHC(O)NH-, -NHC(O)O-, -C(O)OC(O)- oder -C(O)O-, wobei diese Gruppe(n) besonders bevorzugt Bestandteil einer Verbindung aus der jeweils hierfür in Frage kommenden Stoffklasse wie oben erwähnt ist/sind, also Bestandteil eines Kohlenhydrats (Mono-, Di- oder Polysaccharide), einer Aminosäure, eines Peptids oder Proteins, z.B. eines Kollagens oder Enzyms, oder einer Lactam-Struktur der vorgenannten Amino-Verbindungen, eines Polyanhydrids, eines Polyorthoesters, eines Polyethers, eines Polycarbonats, eines Polyamids oder einer Monohydroxysäure (insbesondere einer α- oder ω-Hydroxysäure oder einem Lacton oder Lactid davon) oder eines Oligomeren oder Polymeren der in diesem Absatz genannten Verbindungen ist/sind, wobei diese Stoffe natürlichen oder synthetischen Ursprungs sein können. Der biokompatible, biodegradierbare oder bioresorbierbare Rest wird zusätzlich oder alternativ ausgewählt unter Resten, die unter physiologischen Bedingungen im menschlichen oder tierischen Körper oder durch den Befall von Mikroorganismen gespalten werden können. Günstig sind ferner Aldose- oder Ketosegruppen mit einer Kette von mindestens 4 C-Atomen und mindestens 2 freien oder geschützten Hydroxygruppen. Bevorzugt enthalten die Reste mindestens einen Di- oder Oligosaccharid-, einen Di- oder Oligopeptid-, einen Di- oder Oligoester-, einen Di- oder Oligocarbonsäureamid-, einen Di- oder Oligoguanidino-, einen Di- oder Oligoanhydrid- oder einen Di- oder Oligourethan-Rest. Besonders bevorzugt handelt es sich um Reste mit mindestens einer Kohlehydrat-, Peptid-, Protein-, Polyanhydrid-, Polycarbonat-, Polyorthoester-, Polyether, Polyester-, oder Polyamidgruppe. Jeder der vorgenannten Reste kann eine einzelne, mehrere identische oder eine Kombination von mehreren unterschiedlichen der vorgenannten Gruppen aufweisen. Das Material kann identische oder unterschiedliche Reste enthalten.

Der Ausdruck "oligomer" wird für 2 bis 5 aufeinander folgende, verwandte Struktureinheiten verwendet; eine Molekülstruktur mit 6 oder mehr solchen Struktureinheiten wird mit "polymer" bezeichnet.

Um strukturierbare biodegradierbare Trägermaterialien zu erhalten, müssen die o. g. physiologisch degradierbaren Strukturelemente funktionalisiert werden, so dass eine gemeinsame Vernetzungsreaktion, z. B. eine Polymerisation oder eine Polykondensation und damit eine durch äußere Einflüsse initiierte Vernetzung durch photolithographische Prozesse ermöglicht wird.

Die Zwei- oder Mehrphotonenpolymerisation des organischen, über 2-Photonen- oder Mehrphotonen-Polymerisation polymerisierbaren Rests kann über eine bzw. mehrere Gruppen erfolgen, die sich radikalisch polymerisieren lassen. Zwar sind auch solche Systeme geeignet, die sich mit Hilfe eines kationischen UV-Starters polymerisieren lassen, beispielsweise Epoxysysteme (siehe z.B. C.G. Roffey, Photogeneration of Reactive Species for UV Curing, John Wiley & Sons Ltd, (1997)). Doch neigen diese Systeme zu parasitärer Polymerisation, d.h. es findet auch eine Polymerisation in die nicht belichteten Bereiche hinein statt, weswegen sie für Anwendungsbereiche mit extremen Anforderungen an die Feinheit und Glätte der Oberflächen, z.B. hochaufgelöste Lithographie, weniger gut geeignet sein können (je nach Modifikation). Als radikalisch polymerisierbare Gruppen eignen sich nichtaromatische C=C-Doppelbindungen wie Allyl- oder Vinylgruppen, besonders bevorzugt sind allerdings einer Michael-Addition zugängliche Doppelbindungen. Acrylat- und Methacrylatreste sind ganz besonders bevorzugt. Nachstehend wird häufig der Ausdruck (Meth)Acryl... verwendet, um Acrylsäure(derivate) und/oder Methacrylsäure(derivate) alleine oder in Kombination zu bezeichnen. Es sei darauf hingewiesen, dass (Meth)Acrylatreste nicht als biokompatibel, biodegradierbar oder bioresorbierbar anzusehen sind, auch wenn sie über eine Estergruppe an den Rest des jeweiligen Moleküls angebunden sind.

Für die Polymerisationsreaktion kann ein rein organisches Material eingesetzt werden, das die erfindungsgemäßen Bestandteile aufweist: Das genannte Material kann mindestens eine Verbindung enthalten, die sowohl einen organischen, über 2-Photonen- oder Mehrphotonen-Polymerisation polymerisierbaren Rest als auch eine biokompatible, biodegradierbare oder bioresorbierbare Gruppe.

Die Erfindung offenbart in diesem Zusammenhang Funktionalisierungsreaktionen (potentiell) physiologisch degradierbarer Stoffe und Materialklassen, deren Produkte in Hinblick auf die mögliche Toxizität unbedenklicher sind als die im Stand der Technik beschriebenen. Es handelt sich dabei um die Verknüpfung von hydroxygruppenhaltigen Ausgangsmaterialien, insbesondere von Zuckern, Kohlehydraten, Zuckeralkoholen und gesättigten Hydroxysäuren sowie deren Oligo- und Polymeren, mit Acrylsäure- und Methacrylsäureguppen. Bei den bisher in der Regel genutzten Umsetzungen entstehen toxische Nebenprodukte, die nachteilig sein können, da sie kaum abtrennbar sind und in den dabei erzeugten Gegenständen verbleiben. Erfindungsgemäß werden daher zur Erzeugung der obigen Ausgangsmaterialien Umsetzungen mit (Meth)Acrylsäureestern oder mit (Meth)acrylsäureanhydrid, wobei letzteres teilweise aus dem Stand der Technik bekannt ist, eingesetzt. Die Erfindung stellt dementsprechend ein in den Ansprüchen definiertes Verfahren zum Herstellen einer Verbindung mit der Formel (A)

R'-CHR"-O-C(O)-CR‴=CH₂ (A)

bereit, worin der Rest R‴ H oder CH₃ bedeutet und worin R'CHR" abgeleitet ist von einer Verbindung R'-CHR"OH, ausgewählt unter C-6-Pyranosen oder Furanosen oder davon abgeleitete reine oder gemischte Di- oder Polysaccharide, in denen pro Zuckermolekül zweimal jeweils zwei Hydroxygruppen durch eine Isopropylidengruppe geschützt sind, Milchsäure, Polylactate und Polymere von γ.-Butyro-, Valerio- oder Caprolacton, dadurch gekennzeichnet, dass eine Verbindung

R'-CHR"OH

mit einer Verbindung

R^{IV}O-C(O)-CR‴=CH₂,

worin R^{IV} ein Alkylrest mit vorzugsweise 1 bis 20, stärker bevorzugt mit 1 bis 10 Kohlenstoffatomen, der mit einer oder mehreren Gruppen O-C(O)-CR‴=CH₂ substituiert sein kann und vorzugsweise CH₃, C₂H₅ oder CH₂-CH₂-O-C(O)-CR‴=CH₂ ist, unter Bedingungen umgesetzt wird, die das Reaktionsgleichgewicht zum Produkt hin verschieben. Als Bedingung, die das Reaktionsgleichgewicht zum Produkt hin verschiebt, ist vorzugsweise ein großer Überschuss an (Meth)Acrylsäureester zu nennen, die gleichzeitig als Lösungsmittel fungieren kann. Stattdessen oder zusätzlich kann ein Katalysator verwendet werden, beispielsweise ein Alkoxytitanat. Im erfindungsgemäßen Verfahren eingesetzte Ausgangsmaterialien sind C-6-Pyranosen oder Furanosen oder davon abgeleitete reine oder gemischte Di- oder Polysaccharide, in denen pro Zuckermolekül zweimal jeweils zwei Hydroxygruppen durch eine Isopropylidengruppe geschützt sind, Milchsäure, Polylactate und Polymere von γ.-Butyro-, Valerio- oder Caprolacton. Überraschenderweise hat sich dabei herausgestellt, dass bei Verwendung von Polylactonen je nach Reaktionsführung die Polymeren unter Umesterung mit dem (Meth)Acrylester gespalten werden, so dass man in manchen Fällen monomere Produkte der Formel (A) aus polymeren Ausgangsmaterialien erhält.

Bei den oben beschriebenen, erfindungsgemäßen Funktionalisierungsreaktionen werden insbesondere toxische Funktionalisierungsstoffe vermieden. Bei den Produkten handelt es sich um Zucker, Kohlehydrate, Polyether oder Polyester, die eine definierte Länge aufweisen können und durch Mehr-Photonen-Prozesse vernetzbar und somit strukturierbar sind.

Das Badmaterial kann weiterhin Gruppen oder Reste enthalten, die einer anorganischen Vernetzung zugänglich sind oder bereits anorganisch vernetzt sind, darunter insbesondere oligomerisierte oder stärker polymerisierte Einheiten mit -O-Si-O- oder -O-M-O-Bindungen mit M gleich Bor, Aluminium oder einem Übergangsmetall.

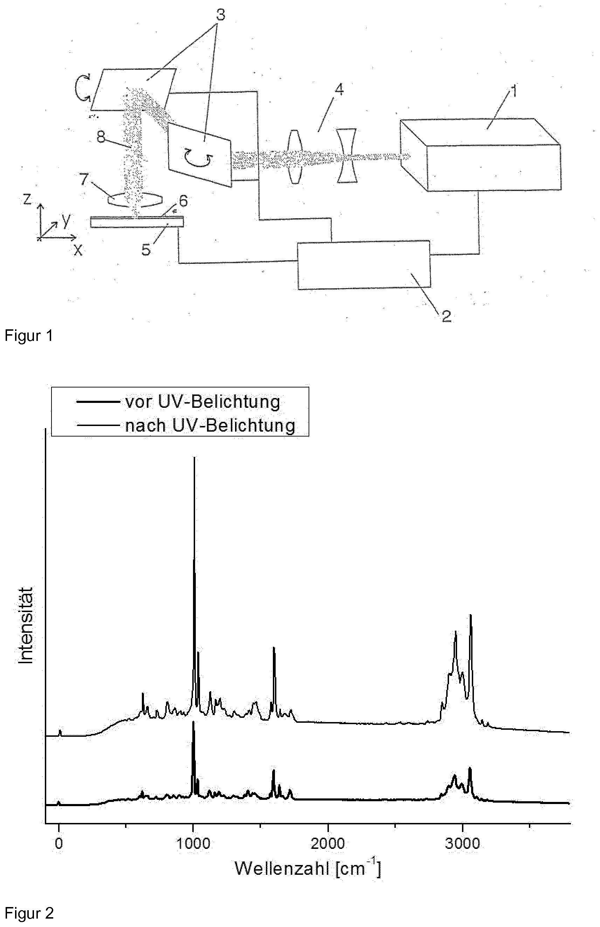

**Figur 1** zeigt beispielhaft eine Anordnung zur Erzeugung dreidimensionaler Strukturen: Als Strahlquelle (1) dient ein Laser, z.B. ein gepulster Laser für die Erzeugung ultrakurzer Laserpulse (Pulsdauer 10 ps bis 10 fs). Um die Qualität des Laserfokus zu kontrollieren, können strahlformende Optiken (4) zum Einsatz kommen (z.B. eine Strahlaufweitung für einen kleinstmöglichen Fokusdurchmesser). Zwei computergesteuerte, rotierende Spiegel (3) werden eingesetzt, um den Laserstrahl in definierter Weise in x- und y-Richtung abzulenken. Der Laserstrahl (8) wird mit Hilfe einer fokussierenden Optik (7) in das zu polymerisierende Harz (5) fokussiert. Bei dieser Optik handelt es sich um eine einzelne Linse oder um Linsensysteme, z.B. Mikroskop-Optiken. Das zu polymerisierende Harz befindet sich unter oder auf einem transparenten Substrat (6). Das Harz kann entweder durch das Substrat (6) bestrahlt werden (siehe Skizze), das in diesem Falle eine hohe optische Oberflächengüte aufweisen sollte, oder es kann sich auf der Oberseite des Substrates befinden und direkt bestrahlt werden (ohne Abbildung). Das Substrat mit dem Harz kann mit Hilfe von computergesteuerten Positioniersystemen in x-, y- und z-Richtung in definierter Weise bewegt werden. Dies, zusammen mit den rasternden Spiegeln, ermöglicht es, den Laserfokus in drei Dimensionen durch das Harz bzw. das Harz durch den Fokus zu bewegen. Alternativ kann die fokussierende Optik (7) in z-Richtung bewegt werden, um den Fokus in z-Richtung durch das Harz zu bewegen. Die Komponenten Laser (1), Scanner (3), sowie alle Positioniereinheiten werden von einem PC (2) gesteuert und kontrolliert.

Die Erfindung soll nachstehend anhand von Ausführungsbeispielen näher erläutert werden.

### Allgemeines Beispiel (nur Referenzbeispiel) Herstellung eines Badmaterials aus hydrolytisch kondensierten Silanen und acrylierten oder methacrylierten organischen Verbindungen

Aus Methacryloxypropyl-trimethoxisilan und Diphenylsilandiol im Molverhältnis 1:1 wurde ein anorganisch-organisches Hybridpolymer hergestellt wie in WO 01/04186 A1 beschrieben. Als Katalysator für die hydrolytische Kondensation diente Ba(OH)₂ oder auch Ba(OH)₂·8H₂O.

Die wie unten in den Beispielen 1 bis 5 hergestellten acrylierten und methacrylierten Zielverbindungen wurden in unterschiedlichen Molverhältnissen (3:1, 1:1, 1:2 und 1:3) mit diesem Hybridpolymer versetzt und bis zu 30 Minuten im Ultraschallbad bei 45 kHz behandelt. Anschließend wurden 2 Gew.-% Photoinitiator (Irgacure^{®} 369, Irgacure^{®} Oxe01, Irgacure^{®} Oxe02 oder dgl.) zugegeben und dieser für weitere bis zu 30 Minuten im Ultraschallbad bei 45 kHz gelöst.

### Allgemeines Beispiel (nur Referenzbeispiel) Strukturierung mit Hilfe von Mehrphotonenpolymerisation

Das voranstehend beschriebene Hybridpolymer ist bereits anorganisch vernetzt. Durch die nachstehend näher erläuterte Strukturierung mittels TPA (Mehrphotonen-Polymerisation) werden die genannten Zielverbindungen zusammen mit dem Hybridpolymer weiter organisch vernetzt, sodass neue Hybridpolymere mit anorganischen und organischen Vernetzungskomponenten entstehen.

### Allgemeines Beispiel (nur Referenzbeispiel) Degradationstudie

Einige Materialformulierungen wurden unter Bestrahlung durch UV-Licht der Wellenlängen 254 nm und 366 nm für 1 h in einer Teflonform ausgehärtet. Die so entstandenen Stäbchen (25 × 2 × 2 mm³) wurden gewogen, in 1,5 ml PBS-Lösung (phosphatgepufferte physiologische Salzlösung mit einem pH-Wert von annähernd 7,4) eingelegt und jeweils nach 1 d, 3 d, 7 d, 14 d, 21 d und 35 d und anschließend alle 28 Tage entnommen. Die Proben wurden mit Reinstwasser abgespült, für 1 h bei 110°C getrocknet und 30 min bei Zimmertemperatur zum Auskühlen stehen gelassen. Anschließend wurden die Stäbchen nochmals gewogen und der prozentuale Gewichtsverlust in Abhängigkeit der Zeit bestimmt. Die PBS-Lösung wurde im Schnitt alle 3 Tage ausgewechselt, um eine Sättigung der Lösung zu verhindern. Bei jeder Entnahme und jedem Lösungswechsel wurde der pH-Wert gemessen, um so Hinweise auf freigesetzte Säuren zu erhalten.

### Beispiel 1 (nur Referenzbeispiel) Funktionalisierung von 1,2:5,6-Di-O-isopropyliden-α-D-glucofuranose mit Acrylsäureethylester und 2-Photonenpolymerisation unter Verwendung des Produkts

### 1.1 Herstellung von (0,2 mmol) 6-O-Acroyl-1,2:5,6-Di-O-isopropyliden- α-D-glucofuranose

In einem 50 ml Schlenckkolben mit Mikrodestille wurden unter Stickstoff-Atmosphäre 0,60 g (4,83 mmol) para-Methoxyphenol, 2,93 g (11,3 mmol) 1,2:5,6-Di-*O*-isopropyliden-α-D-glucofuranose, 18 ml (25,3 g, 282,74 mmol) Acrylsäureethylester und 0,60 g (2,03 mmol) Tetra(isopropyloxy)titan gegeben und die rote Reaktionslösung auf 112°C erhitzt. Nach 40 Minuten wurde bei 74-75°C ein farbloses Acrylsäureethylester-Ethanol-Azeotrop abdestilliert. Nach zwei Stunden wurde die Lösung kurz an Luft abgekühlt und 11 ml Petrolether (Sdp. 30-50°C) und 2,2 ml Wasser zugegeben, wobei über Nacht ein gelber Niederschlag ausfiel. Der Niederschlag wurde 10 Minuten bei 4000 U·min⁻¹ abzentrifugiert, zwei Mal mit je 15-20 ml Pentan gewaschen und erneut abzentrifugiert. Die organischen Lösungen wurden vereinigt und destillativ entfernt bis ein blass gelber Feststoff zurück blieb. Dieser Feststoff wurde erneut in etwa 30 ml Petrolether gelöst und drei Mal mit je 27 ml Wasser, drei Mal mit je 0,5 M Natronlauge gewaschen. Das Lösungsmittel wurde erneut destillativ entfernt und der blass gelbe bis weiße Feststoff im Ölpumpenvakuum getrocknet.

Ausbeute: 1,01 g (3,21 mmol, 28,4%)

¹H-NMR (CDCl₃, 23°C, 400,1 MHz, [ppm]): δ =1,30 (s, 3 H, C*H₃*), 1,41 (s, 3 H, C*H₃*), 1,52 (s, 3 H, C*H₃*), 4,05 (m, 2 H, C*H₂*), 4,24 (m, 2 H, C*H* - C4 + C5), 4,53 (d, ³J_{H,H} = 3,79 Hz, 1 H, C*H-*C2), 5,33 (d, ³J_{H,H} = 1,77 Hz, 1 H, C*H* - C3), 5,90 (m, 2 H, C*H* - C1, C=OCH=C*H₂ cis*), 6,13 (dd, ³J_{H,H} = 10,48 Hz, ³J_{H,H} = 17,31 Hz, 1 H, C=OC*H*=CH₂), 6,45 (d, ³J_{H,H} = 17,43 Hz, 1 H, C=OCH=C*H₂ trans*). - ¹³C-NMR (CDCl₃, 23°C 100,6 MHz, [ppm]): δ = 25,20, 26,17, 26,69, 26,80 (CH₃), 67,09 (*C*H₂), 72,40 (CH - C5), 76,18 (CH - C3), 79,72 (CH - C4), 83,26 (CH - C2), 105,03 (CH - C1), 109,31 (*C_{q}* - C5 + C6), 112,29 (*C_{q}* - C1 + C2), 127,68 (C=O*C*H=CH₂), 131,98 (C=OCH=*C*H₂), 164,69 (*C*=OCH=CH₂).

FT-IR (ATR, 23 °C, [cm⁻¹]): *υ̃* = 1729 (s, v(C=O)), 1635 (m, v(C=C)), 1157 (s, v(C-O)), 1013 (ss, v C-O-C)), 802 (s, δ(CH₂)).

µ-Raman (23°C, [cm⁻¹]): *υ̃* =2947 (s, v(C-H_{2/3})), 1735 (m, v(C=O)), 1639 (m, v (C=C)), 1441 (m, v C-H_{2/3})), 802 (s, δ(CH₂)).

### 1.2 Herstellung eines Badmaterials unter Verwendung der gemäß 1.1 hergestellten (0,2 mmol) 6-O-Acroyl-1,2:5,6-Di-O-isopropyliden-α-D-glucofuranose und eines anorganisch-organischen Hybridpolymers

In einem 5 ml Rollrandglas wurden 63,6 mg (0,2 mmol) 6-O-Acroyl-1,2:5,6-Di-*O*-isopropyliden-α-D-glucofuranose und 540 mg (1,4 mmol) eines anorganisch-organischen Hybridpolymers aus 3-Methacryloxypropyl-trimethoxisilan und Diphenylsilandiol in einer 1:1-Stöchiometrie, das unter Katalyse von Ba(OH)₂ hydrolytisch kondensiert worden war, gegeben, worauf die Mischung 5 h bei 50°C im Ultraschallbad bei 45 kHz behandelt wurde. Es entstand eine feine weißlich trübe Dispersion. Diese Dispersion wurde nach Zugabe von 12 mg (0,03 mmol, 2 Gew.-%) Irgacure 369 10 Minuten bei 45 kHz im Ultraschallbad behandelt.

### 1.3 Herstellung eines Formkörpers

Die erhaltene Suspension wurde mittels Zwei-Photonen-Absorption (TPA) organisch polymerisiert und strukturiert wie oben im allgemeinen Beispiel "Mehrphotonenpolymerisation" angegeben.

### Beispiel 2 (nur Referenzbeispiel) Funktionalisierung von 1,2:5,6-Di-O-isopropyliden-α-D-glucofuranose mit Methacrylsäureanhydrid und Herstellung von Formkörpern unter Verwendung des Produkts

### 2.1 Herstellung von (3-Methacroyl)-1,2:5,6-di-(O)-isopropyliden-α-D-glucofuranose

In einem 100 ml Schlenckkolben mit Rückflusskühler wurden unter Stickstoff-Atmosphäre 2,95 g (11,3 mmol) 1,2:5,6-Di-O-isopropyliden-α-D-glucofuranose in 15 ml trockenem Pyridin gelöst und 3 ml (3,13 g, 20,28 mmol) Methacrylsäureanhydrid zugegeben. Die Reaktionslösung wurde 3,5 h auf 65 °C erhitzt und anschließend wurden 16 ml Wasser zugegeben. Die Reaktionslösung wurde eine weitere Stunde auf 65°C und anschließend auf 30°C für 17 h erhitzt, wobei die klare farblose Lösung milchig trüb wurde. Die Reaktionslösung wurde mit 3 ml, 6 ml und 10 ml Petrolether extrahiert. Die vereinigten organischen Lösungen wurden drei Mal mit je 15 ml Wasser und drei Mal mit 14 ml 5%iger Natriumhydrogencarbonat-Lösung gewaschen. Das Lösungsmittel wurde destillativ entfernt und ein weißer Feststoff erhalten. Dieser Feststoff wurde mit wenig Ethanol-Wasser-Lösung (14:10) gewaschen und im Ölpumpenvakuum getrocknet.

Ausbeute: 1,70 g (5,18 mmol, 45,7%)

¹H-NMR (CDCl₃, 23°C, 400,1 MHz, [ppm]): δ =1,29 (s, 6 H, C*H₃*), 1,40 (s, 3 H, C*H₃*), 1,52 (s, 3 H, C*H₃*), 1,94 (t, ³J_{H,H} = 1,52 Hz, 3 H, C=OC*C*H₃=CH₂), 4,04 (m, 2 H, C*H₂*), 4,25 (m, 2 H, C*H-*C4 + C5), 4,52 (d, ³J_{H,H} = 3,52 Hz, 1 H, C*H* - C2), 5,28 (d, ³J_{H,H} = 2,28 Hz, 1 H, C*H* - C3), 5,61 (t, ³J_{H,H} = 1,52 Hz, 1 H, C=OCCH₃=C*H₂ cis),* 5,88 (d, ³J_{H,H} = 3,8 Hz, 1 H, CH-C1), 6,11 (s, 1 H, C=OCH=C*H₂ trans*). - ¹³C-NMR (CDCl₃, 23°C 100,6 MHz, [ppm]): δ = 18,23 (C=OC*C*H₃=CH₂), 25,18, 26,15, 26,69, 26,77 (CH₃), 67,20 (*C*H₂), 72,49 (CH - C5), 76,40 (CH - C3), 79,87 (CH - C4), 83,24 (CH - C2), 105,03 (CH - C1), 109,28 (*C_{q}* - C5 + C6), 112,23 (*C_{q}* - C1 + C2), 126,51 (C=OC*C*H₃=CH₂), 135,78 (C=OC*C*H₃=CH₂), 165,92 (*C*=OCH=CH₂).

FT-IR (ATR, 23°C, [cm⁻¹]): *υ̃* = 1716 (s, v(C=O)), 1631 (w, v(C=C)), 1159 (s, v(C-O)), 1021 (ss,v(C-O-C)).

µ-Raman (23°C, [cm⁻¹]): *υ̃* = 2947 (s, v(C-H_{2/3})), 1735 (m, v(C=O)), 1639 (m, v(C=C)), 1441 (m, v(C-H_{2/3}), 802 (s, δ(CH₂)).

### 2.2 Herstellung eines härtbaren Harzes unter Verwendung von (3-Methacroyl)-1,2:5,6-di-(O)-isopropyliden-α-D-glucofuranose

Die entstandene (3-Methacroyl)-1,2:5,6-di-(O)-isopropyliden-α-D-glucofuranose wurde analog zu Beispiel 1.2 mit einem Hybridpolymer aus 3-Methacryloxypropyl-trimethoxysilan und Diphenylsilandiol zusammengegeben und behandelt, wobei die Bestandteile im molaren Verhältnis von 33% modifiziertem Zucker und 67% Hybridpolymer eingesetzt wurden.

### 2.3 Polymerisation des Harzes unter UV-Bestrahlung

Das gemäß 2.2 hergestellte Harz wurde in eine Form gefüllt und mit Hilfe von UV-Belichtung organisch polymerisiert (ausgehärtet). Das µ-Raman-Spektrum vor und nach der Polymerisation ist in **Figur 2** dargestellt. Man erkennt eine deutliche Abnahme der C=C-Bande bei 1639 cm⁻¹ im Verhältnis zur C=O-Bande bei 1718 cm⁻¹ als Folge der organischen Vernetzung.

### 2.4 Herstellung eines Formkörpers mittels 2-Photonen-Absoprtion

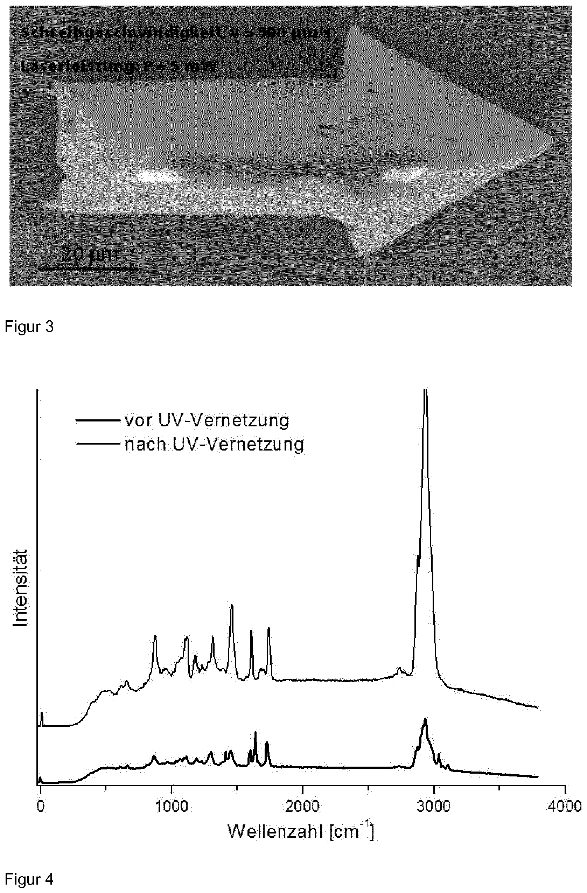

Das gemäß 2.2 hergestellte Harz wurde mittels Zwei-Photonen-Absorption (TPA) organisch polymerisiert und strukturiert wie oben im allgemeinen Beispiel "Mehrphotonenpolymerisation" angegeben. Ein auf diese Weise hergestellter Formkörper ist in **Figur 3** dargestellt.

### Beispiel 3 (nur Referenzbeispiel) Funktionalisierung von Polycaprolacton-diol mit Acrylsäureethylester, Herstellung von Formkörpern unter Verwendung des Produkts und Degradationsstudie dazu

### 3.1 Herstellung von (6-Acroyl)hexansäureethylester

In einem 100 ml Schlenckkolben mit Mikrodestille wurden unter Stickstoff-Atmosphäre 9,00 g (16,98 mmol) Polycaprolacton-diol, 26,5 ml (37,2 g, 372 mmol) Acrylsäureethylester, 0,85 g (6,85 mmol) para-Methoxyphenol und 0,88 ml (0,84 g 2,94 mmol) Tetra(isopropyloxy)-titan gegeben und die rote Reaktionslösung auf 113°C erhitzt. Nach 40 Minuten wurde bei 74-75°C ein farbloses Acrylsäureethylester-Ethanol-Azeotrop abdestilliert. Nach 2 Stunden wurde die Lösung für 30 min an Luft abgekühlt und 16,1 ml Petrolether (Sdp. 30-50°C) und 3,2 ml Wasser zugegeben, wobei nach 4,5 h ein gelber Niederschlag ausfiel. Der Niederschlag wurde nach weiteren 15 h 10 Minuten bei 4000 U·min⁻¹ abzentrifugiert, zwei Mal mit Pentan gewaschen und erneut abzentrifugiert. Die organischen Lösungen wurden vereinigt und drei Mal mit je 40 ml Wasser gewaschen. Das Lösungsmittel wurde destillativ entfernt, sodass eine gelbe Lösung zurückblieb. Diese Lösung wurde bei 7·10⁻³ mbar und 50-62°C fraktioniert destilliert, wobei eine farblose Flüssigkeit erhalten wurde. Diese farblose Flüssigkeit wurde anschließend noch säulenchromatographisch gereinigt (Eluens: n-Pentan:Essigsäureethylester = 10:1).

Ausbeute: 3,41 g (5,34 mmol, 31,4%)

¹H-NMR (CDCl₃, 23°C, 400,1 MHz, [ppm]): δ = 1,19 (t, ³*J*_{HH} = 7,32 Hz, 3 H, OCH₂C*H₃*), 1,36 (m, 2 H, OCH₂CH₂C*H₂*CH₂CH₂C=O), 1,62 (m, 4 H, OCH₂C*H₂*CH₂C*H₂*CH₂C=O), 2,24 (t, ³*J*_{HH} =

7,56 Hz, 2 H, OCH₂CH₂C*H₂*CH₂CH₂C=O), 4,07 (m, 4 H, OC*H₂*CH₂CH₂CH₂CH₂C=OOC*H₂*CH₃), 5,75 (dd, ²*J*_{HH} = 1,52 Hz, ³*J*_{HH} = 10,36 Hz, 1 H, OC=OCH=C*H₂* cis), 6,05 (dd, ³*J*_{HH} = 10,36 Hz, ³*J*_{HH} = 17,43 Hz, 1 H, OC=OC*H*=CH₂), 6,33 (dd, ²*J*_{HH} = 1,52 Hz, ³*J*_{HH} = 17,43 Hz, OC=OCH=C*H₂* trans). - ¹³C-NMR (CDCl₃, 23 °C 100,6 MHz, [ppm]): δ = 13,27 (OCH₂*C*H₃), 23,57, 24,51, 27,30, 33,15, 59,24, 63,32 (*C*H₂), 127,52 (OC=O*C*H=CH₂), 129,54 (OC=OCH=*C*H₂), 165,25, 172,51 (C=O).

FT-IR (ATR, 23°C, [cm⁻¹]): *υ̃* = 3100-3000 (w, v(=C-H)), 2945, 2866 (m, v(-C-H)), 1722 (s, v(-C=O)), 1636, 1620 (w, v(-C=C)), 1511, 1457, 1408 (m, δ(-CH₂)), 1374, 1270, 1186, 1098, 1061, 1035 (s, v(-C-O-C)).

µ-Raman (23°C, [cm⁻¹]): *υ̃* = 1072, 1199, 1304 (v(-C-O-C)), 1412, 1453 (δ(CH₂)), 1639 (v(C=C)), 1726 (v(-C=O)), 2939 (v(-C-H)), 3042, 3072, 3109 (v(=C-H)).

### 3.2. Polymerisation von (6-Acroyl)hexansäureethylester

Der gemäß 3.1. hergestellte (6-Acroyl)hexansäureethylester wurde mit 2 Gew.-% Irgacure^{®} 369 als Photostarter versetzt und durch die Belichtung mit UV-Licht vernetzt. Das µ-Raman-Spektrum vor und nach der Polymerisation ist in **Figur 4** dargestellt. Man erkennt eine deutliche Abnahme der C=C-Bande bei 1634 cm⁻¹ im Verhältnis zur C=O-Bande bei 1738 cm⁻¹ als Folge der organischen Vernetzung.

### 3.3 Herstellung eines Formkörpers aus (6-Acroyl)hexansäureethylester mittels 2-Photonen-Absorption

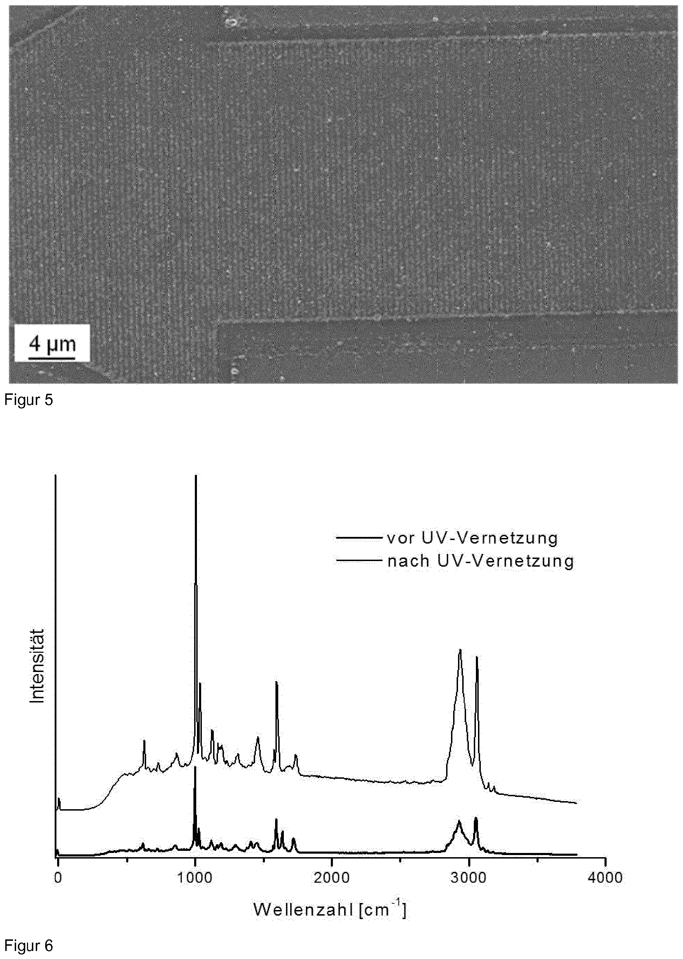

Der gemäß 3.1. hergestellte (6-Acroyl)hexansäureethylester wurde mit 2 Gew.-% Irgacure^{®} 369 als Photostarter versetzt und mittels Zwei-Photonen-Absorption (TPA) organisch polymerisiert und strukturiert wie oben im allgemeinen Beispiel "Mehrphotonenpolymerisation" angegeben. Ein auf diese Weise hergestellter Formkörper ist in **Figur 5** dargestellt.

### 3.4 Herstellung eines härtbaren Harzes unter Verwendung des gemäß 3.1 hergestellten (6-Acroyl)hexansäureethylesters und eines anorganisch-organischen Hybridpolymers

Der (6-Acroyl)hexansäureethylester wurde analog zu Beispiel 1.2 mit einem Hybridpolymer aus 3-Methacryloxypropyl-trimethoxysilan und Diphenylsilandiol zusammengegeben und behandelt, wobei die Bestandteile im molaren Verhältnis von 50% (6-Acroyl)hexansäureethylester und 50% Hybridpolymer eingesetzt wurden.

### 3.5 Polymerisation des gemäß 3.4. erhaltenen Harzes unter UV-Bestrahlung

Das gemäß 3.4 hergestellte Harz wurde in eine Form gefüllt und mit Hilfe von UV-Belichtung organisch polymerisiert (ausgehärtet). Das µ-Raman-Spektrum vor und nach der Polymerisation ist in **Figur 6** dargestellt. Man erkennt eine deutliche Abnahme der C=C-Bande bei 1639 cm⁻¹ im Verhältnis zur C=O-Bande bei 1718 cm⁻¹ als Folge der organischen Vernetzung.

### 3.6 Herstellung eines Formkörpers mittels 2-Photonen-Absoprtion

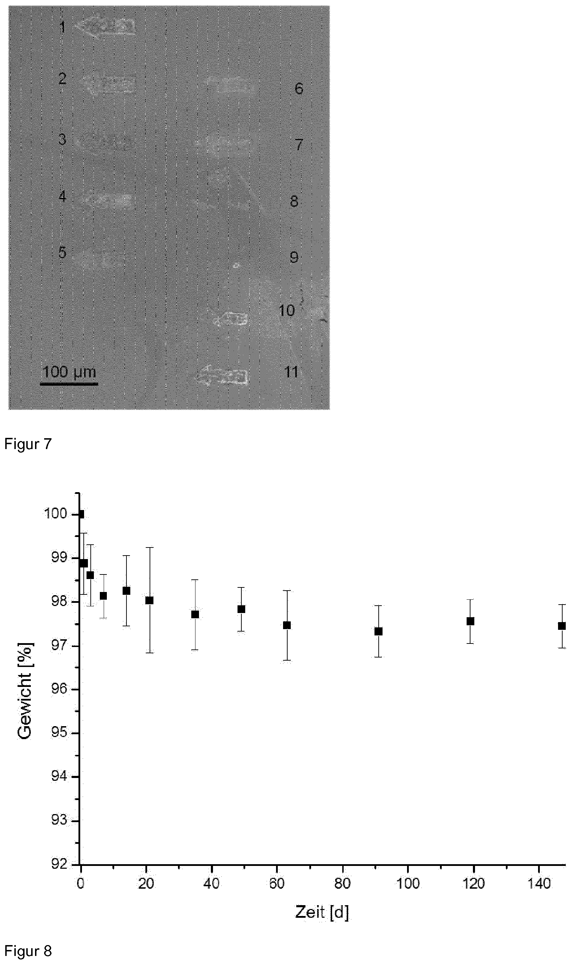

Das gemäß 3.4 hergestellte Harz wurde mittels Zwei-Photonen-Absorption (TPA) organisch polymerisiert und strukturiert wie oben im allgemeinen Beispiel "Mehrphotonenpolymerisation" angegeben. Auf diese Weise hergestellte Formkörper (strukturiert aus einem Tropfen auf einer Glasplatte) sind in **Figur 7** dargestellt.

### 3.7 Degradationsstudie

Das gemäß 3.4 hergestellte Harz wurde wie im allgemeinen Beispiel erläutert ausgehärtet und danach in PBS-Lösung eingelegt. Das Ergebnis ist graphisch in **Figur 8** dargestellt. In einem Vergleichsversuch mit dem anorganisch-organischen Hybridpolymer ohne (6-Acroyl)hexansäureethylester konnte keinerlei Degradation beobachtet werden.

### Beispiel 4 (nur Referenzbeispiel) Funktionalisierung von Polycaprolacton-diol mit Methylmethacrylat, Herstellung von Formkörpern unter Verwendung des Produkts sowie Degradations- und Biokompatibilitätsstudien dazu

### 4.1 Herstellung von (6-Methacroyl)hexansäuremethylester

10.0 g (18.9 mmol) Polycaprolactondiol wurden unter Schutzgasatmosphäre in 29.7 mL (276 mmol) Methylmethacrylat gelöst und mit 991 mg (7.98 mmol) *p*-Methoxyphenol sowie 941 mg (3.31 mmol) Tetra(isopropyloxy)-titan(IV) versetzt, woraufhin sich die Lösung dunkelrot verfärbte. Unter Rühren wurde auf 110°C erhitzt und innerhalb von 45 min etwa 4 mL eines Azeotrops aus Methanol und Methylmethacrylat abdestilliert (Übergangstemperatur ca. 45°C). Man ließ die Mischung 30 min lang abkühlen und gab 15.2 mL *n*-Pentan sowie 3.00 mL Wasser hinzu, wodurch ein orangefarbener Niederschlag ausfiel. Dieser wurde durch Zentrifugieren und Abdekantieren entfernt und zweimal mit je etwa 15 mL *n*-Pentan nachgewaschen. Filtrat und Waschlösungen wurden vereinigt und unter vermindertem Druck vom Lösemittel befreit. Der hellgelbe Rückstand wurde durch Destillation bei 6.0 × 10⁻³ mbar aufgereinigt. Siedepunkt: 65°C, Ausbeute: 3.52 g (5.28 mmol, 28%) einer hellvioletten Flüssigkeit. NMR- und UV-Vis-Spektrum zeigten, dass noch Verunreinigungen an *p*-Methoxyphenol enthalten waren, welche durch Säulenchromatographie an einer Kieselgelphase (Eluens: 10:1-Mischung von *n*-Pentan und Essigsäureethylester) entfernt werden konnten.

Ausbeute: 713 mg (3.33 mmol, 8.8%)

¹H-NMR (CDCl₃, 23°C, 400,1 MHz): δ = 1,35 (m, 2 H, OCH₂CH₂C*H₂*CH₂CH₂C=O), 1,62 (m, 4 H, OCH₂C*H₂*CH₂CH₂CH₂C=O), 1,87 (t, ⁴J_{H,H} = 1,26 Hz, 3 H, OC=OCC*H₃*=CH₂), 2,26 (t, 2 H, ³J_{H,H} = 7,45 Hz, OCH₂CH₂C*H₂*CH₂CH₂C=O), 3,60 (s, 3 H, OC*H₃*), 4,08 (t, ³J_{H,H} = 6,57, 2 H, OC*H₂*CH₂CH₂CH₂CH₂C=O), 5,48 (m, 1 H, OC=OCCH₃=C*H₂ cis*), 6,02 (m, 1 H, OC=OCCH₃=C*H₂ trans*). - ¹³C-NMR (CDCl₃, 23°C 100 MHz): δ = 18,46 (OC=OC*C*H₃=CH₂), 24.81, 25.72, 28.45, 34.06 (*C*H₂), 51,67 (OCH₃), 64.58 (*C*H₂), 125.41 (OC=OCCH₃=*C*H₂), 136.59 (OC=O*C*CH₃=CH₂), 167.62, 174.12 (C=O).

FT-IR (ATR, 23°C, [cm⁻¹]) *υ̃* = 3100-3000 (w, v(=C-H)), 2951, 2863 (m, v(-C-H)), 1715 (s, v(-C=O)), 1636 (w, v(-C=C)), 1510, 1438 (m, δ(-CH₂)), 1321, 1296, 1234, 1162, 1101, 1036 (s, v(-C-O-C)).

µ-Raman (23°C, [cm⁻¹]) *υ̃* = 1011, 1050, 1101, 1161, 1178, 1258, 1296 (v(-C-O-C)), 1403, 1448 (v(CH₂)), 1635 (v(C=C)), 1714 (v(-C=O)), 2925 (δ(-C-H)), 3062, 3105 (v(=C-H)).

### 4.2 Herstellung härtbarer Harze unter Verwendung des gemäß 4.1 hergestellten (6-Methacroyl)hexansäuremethylesters

Der (6-Methacroyl)hexansäuremethylester wurde analog zu Beispiel 1.2 mit einem Hybridpolymer aus 3-Methacryloxypropyl-trimethoxysilan und Diphenylsilandiol zusammengegeben und behandelt, wobei die Bestandteile (a) im molaren Verhältnis von 50% (6-Methacroyl)hexansäuremethylesters und 50% Hybridpolymer und (b) von 25% (6-Methacroyl)hexansäuremethylesters und 75% Hybridpolymer eingesetzt wurden.

### 4.3 Polymerisation des gemäß 4.2 erhaltenen Harzes unter UV-Bestrahlung

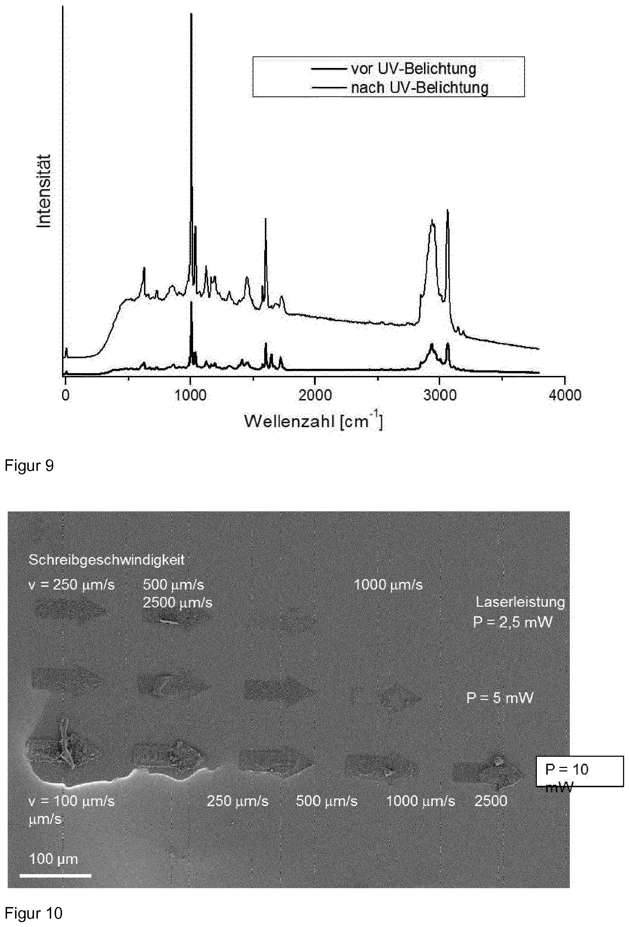

Das gemäß 4.2, Variante (a) hergestellte Harz wurde in eine Form gefüllt und mit Hilfe von UV-Belichtung organisch polymerisiert (ausgehärtet). Das µ-Raman-Spektrum vor und nach der Polymerisation ist in **Figur 9** dargestellt. Man erkennt eine deutliche Abnahme der C=C-Bande bei 1646 cm⁻¹ im Verhältnis zur C=O-Bande bei 1722 cm⁻¹ als Folge der organischen Vernetzung.

### 4.4 Herstellung eines Formkörpers mittels 2-Photonen-Absoprtion

Das gemäß 4.2, Variante (a) hergestellte Harz wurde mittels Zwei-Photonen-Absorption (TPA) organisch polymerisiert und strukturiert wie oben im allgemeinen Beispiel "Mehrphotonen-polymerisation" angegeben. Auf diese Weise hergestellte Formkörper sind in **Figur 10** dargestellt.

### 4.5 Degradationsstudie

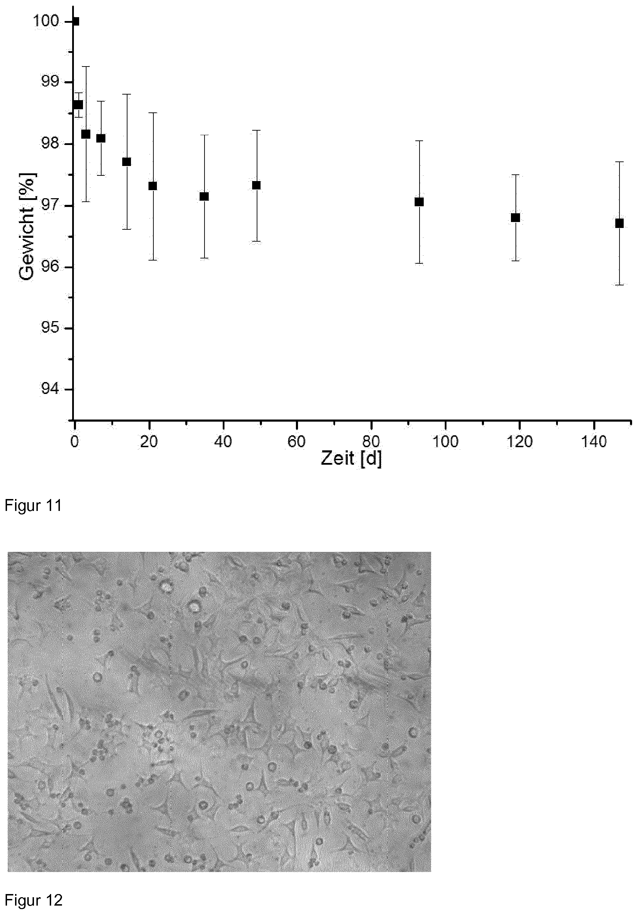

Das gemäß 4.2, Variante (a) hergestellte Harz wurde wie im allgemeinen Beispiel erläutert ausgehärtet und danach in PBS-Lösung eingelegt. Das Ergebnis ist graphisch in **Figur 11** dargestellt.

### 4.6 Biokompatibilitätsstudie

Eine Form mit gemäß 4.2, Variante (b) hergestelltem Harz wurde mit UV-Licht bestrahlt. Nach dem Aushärten wurde ein Biokompatibilitätsversuch mit Mausfibroblasten L929 durchgeführt, die auf die glatte Oberfläche des ausgehärteten Materials aufgebracht wurden. Nach drei Tagen war das Material mit Zellen besiedelt. Diese hatten sich in alle Richtungen ausgestreckt ("Spreading"), wie in **Figur 12** zu erkennen, was ein Zeichen dafür ist, dass es ihnen gut geht.

### Beispiel 5 (erfindungsgemäßes Beispiel) Funktionalisierung von Polycaprolacton-diol mit Di(ethylenglykol)-dimethacrylat und organische Polymerisation des Produkts

### 5.1 Erfindungsgemäße Herstellung von (6-Methacroyl)hexansäure(di(ethylenglykol)methacrylat)ester

In einem 100 ml Schlenckkolben mit Rückflusskühler wurden unter Stickstoff-Atmosphäre 4,72 g (8,90 mmol) Polycaprolacton-diol, 20,0 ml (21,64 g, 89,3 mmol) Di(ethylenglykol)-dimethacrylat und 0,50 ml (0,48 g 1,70 mmol) Tetraisopropyltitan(IV) gegeben und die gelbe Reaktionslösung auf 135°C erhitzt. Nach vier Stunden wurde die Lösung für 30 min an Luft abgekühlt und 10,0 ml Wasser zugegeben, wobei sofort ein gelblicher Niederschlag ausfiel. Der Niederschlag wurde nach weiteren 15 h 10 Minuten bei 4000 U·min⁻¹ abzentrifugiert, mit wenig Pentan gewaschen und erneut abzentrifugiert. Die organischen Lösungen wurden drei Mal mit je 10 ml Wasser gewaschen. Das Lösungsmittel wurde destillativ entfernt, so dass eine farblose Lösung zurückblieb. Diese farblose Flüssigkeit wurde anschließend säulenchromatographisch mit n-Pentan: Essigsäureethylester = 5:1als Eluens gereinigt.

Ausbeute: 5,754 g (16,15 mmol, 45,4%)

¹H-NMR (CDCl₃, 23°C, 400,1 MHz, [ppm]): δ = 1,35 (m, 2 H, C=OOCH₂CH₂C*H₂*CH₂-CH₂C=OO), 1,61 (m, 4 H, C=OOCH₂C*H₂*CH₂C*H*₂CH₂C=OO), 1,86 (s, 3 H, H₂C=CC*H₃*C=OOCH₂CH₂CH₂CH₂CH₂C=O), 1,87 (s, 3 H, OCH₂CH₂OC=OCC*H₃*=CH₂), 2,28 (t, ³J_{H,H} = 7,58 Hz, 2 H, , C=OOCH₂C*H₂*CH₂CH₂CH₂C=OO), 3,64 (t, ³J_{H,H} = 4,80 Hz, 2 H), 3,67 (t, ³J_{H,H} = 4,92 Hz, 2 H, C=OOC*H₂*CH₂OCH₂C*H₂*OC=O), 4,06 (t, ³J_{H,H} = 6,57 Hz, 2 H, C=OOCH₂C*H₂*CH₂CH₂CH₂C=OO), 4,16 (t, ³J_{H,H} = 4,67 Hz, 2 H, C=OOCH₂C*H₂*OCH₂CH₂OC=O), 4,23 (t, ³J_{H,H} = 4,80 Hz, 2 H, C=OOCH2CH2OC*H*2-CH2OC=O), 5,47 (t, ⁴J = 1,52 Hz, 1 H, *H₂*C=CCH₃C=OOCH₂CH₂CH₂CH₂CH₂C=O *cis*), 5,51 (t, ⁴J = 1,52 Hz, 1 H, OCH₂CH₂OC=OCCH₃=C*H₂ cis*), 6,01 (s, 1 H, *H₂*C=CCH₃C=OOCH₂CH₂CH₂CH₂CH₂C=O *trans*), 6,05 (s, 1 H, OCH₂CH₂OC=OC-CH₃=C*H₂ trans*). - ¹³C-NMR (CDCl₃, 23°C 100,6 MHz, [ppm]): δ = 17,31, 17,32 (*C*H₃), 23,51 (C=OOCH₂*C*H₂CH₂CH₂CH₂C=OO), 24,56 (C=OOCH₂*C*H₂CH₂CH₂CH₂C=OO), 27,31 (C=OOCH₂*C*H₂CH₂CH₂CH₂C=OO), 32,98 (C=OOCH₂*C*H₂CH₂CH₂CH₂C=OO), 62,34 (C=OOCH₂CH₂OCH₂CH₂OC=O), 62,75 (C=OOCH₂CH₂O*C*H₂CH₂OC=O), 63,44 (C=OOCH₂*C*H₂CH₂CH₂CH₂C=OO), 68,05, 68,12 ((C=OOCH₂CH₂OCH₂*C*H₂OC=O), 124,28, 124,80 (CCH₃=*C*H₂), 135,11, 135,41 (*C*CH₃=CH₂), 166,27, 166,43 (C=OC*C*H₃=CH₂), 172,43 (C=OOCH₂CH₂CH₂CH₂CH₂*C*=OO).

FT-IR (ATR, 23 °C, [cm⁻¹]): *υ̃* =2951 (m, vₐₛ(C-H)), 2867 (w, vₛ(C-H)), 1715 (s, v(C=O)), 1637 (m, v(C=C)), 1296 (s, v(C-O-C)), 1160 (ss, v(C-O-C)), 940 (s, δ(C-H)), 814 (m, skel. bend). µ-Raman (23 °C, [cm⁻¹]): *υ̃* =868 (m, γ(C-H)), 1444 (m, δ(C-H)), 1639 (m, v(C=C)), 1718 (m, v(C=O)), 2928 (s, vₐₛ(C-H)).

### 5.2 Herstellung eines härtbaren Harzes unter Verwendung von (6-Methacroyl)hexansäure(di(ethylenglykol)methacrylat)ester

(6-Methacroyl)hexan-säure(di(ethylenglykol)methacrylat)ester wurde analog zu Beispiel 1.2 mit einem Hybridpolymer aus 3-Methacryloxypropyl-trimethoxysilan und Diphenylsilandiol zusammengegeben und behandelt, wobei die Bestandteile im molaren Verhältnis von 50% Methacrylatester und 50% Hybridpolymer eingesetzt wurden.

### 5.3 Polymerisation des Harzes unter UV-Bestrahlung

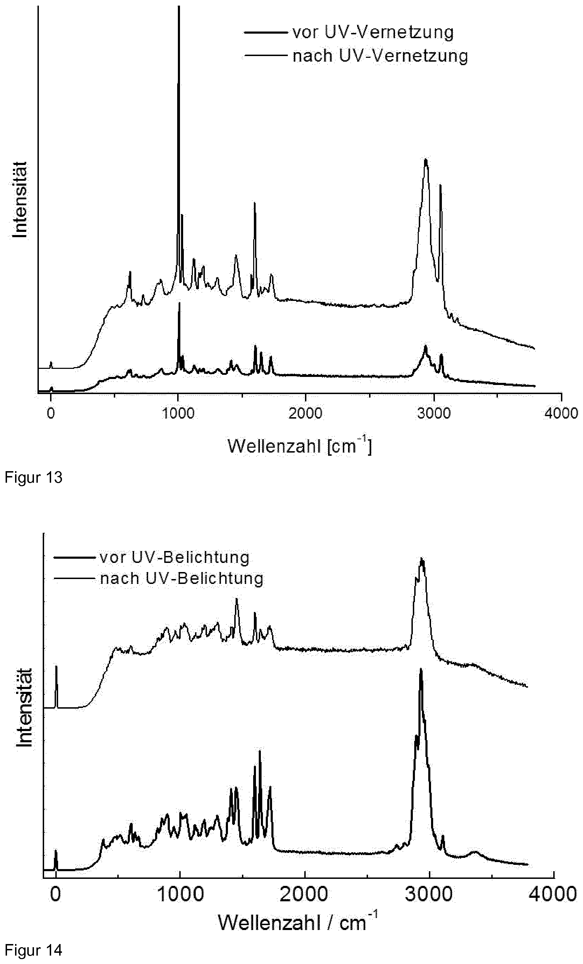

Das gemäß 5.2 hergestellte Harz wurde in eine Form gefüllt und mit Hilfe von UV-Belichtung organisch polymerisiert (ausgehärtet). Das µ-Raman-Spektrum vor und nach der Polymerisation ist in **Figur 13** dargestellt. Man erkennt eine deutliche Abnahme der C=C-Bande bei 1648 cm⁻¹ im Verhältnis zur C=O-Bande bei 1722 cm⁻¹ als Folge der organischen Vernetzung.

### Beispiel 6 (nur Referenzbeispiel) Funktionalisierung von 1,2:5,6-Di-O-isopropyliden-D-mannitol mit Methacrylsäureanhydrid

In einen 100 mL Kolben mit aufgesetztem Rückflusskühler wurden 3,01 g (11,5 mmol) 1,2:5,6-di-O-isopropyliden-D-mannitol, sowie 15,0 mL Pyridin gegeben und bis zur vollständigen Auflösung des Mannitols gerührt. Anschließend wurden 3,00 mL (3,13 g, 20,3 mmol) Methacrylsäureanhydrid hinzugegeben und die Lösung weitere dreieinhalb Stunden bei 65°C unter Rückfluss gerührt. Danach wurden 16,2 mL Wasser hinzugegeben und die farblose Lösung für eine weitere Stunde bei 65°C weiter gerührt. Die Mischung wurde auf 30°C abgekühlt und 17 h gerührt. Nach diesen 17 h wurde die Lösung drei Mal mit je 10,0 mL Pentan extrahiert. Die organischen Phasen wurden vereinigt, drei Mal mit je 20 mL Wasser und drei Mal mit je 20 mL 5%iger Natriumhydrogencarbonat-Lösung gewaschen und über Natriumsulfat getrocknet. Nach Abfiltrieren des Trockenmittels erhielt man eine klare, farblose Flüssigkeit. Das Lösungsmitten wurde abdestilliert und das Produkt im Ölpumpenvakuum getrocknet, worauf ein farbloser Feststoff erhalten wurde.

### Beispiel 7 (nur Referenzbeispiel) Synthese von O-(Methacryloxyethyl)-N-(triethoxysilylpropyl)urethan, Herstellung von Formkörpern unter dessen Verwendung sowie Degradations- und Biokompatibilitätsstudien dazu

### 7.1. Synthese von O-(Methacryloxyethyl)-N-(triethoxysilylpropyl)urethan

247 g (1 mol) (3-isocyanatopropyl)triethoxysilan wurden langsam zu einer Mischung aus 130 g (1 mol) (Hydroxyethyl)methacrylat, 150 mg Hydroquinonmonomethylether und 180 mg Dibutyl-Zinn-Dilaureat gegeben und die Lösung für 24 Stunden bei Raumtemperatur gerührt. Anschließend war die Isocyanat-IR-Bande bei 2245 cm⁻¹ nicht mehr zu beobachten. Das Produkt hat die folgende Strukturformel:

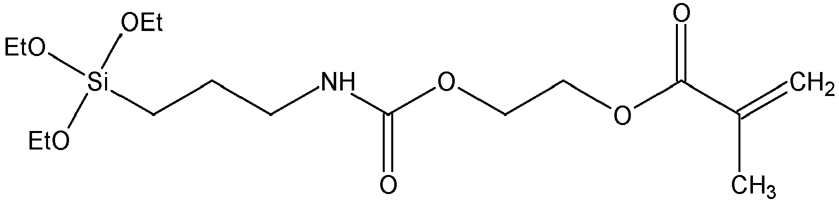

Dieses Molekül bietet die Möglichkeit zur organischen-photochemischen Vernetzung an der Methacrylatgruppe, kann durch die Alkoxysilan-Einheit ein anorganisches Netzwerk bilden und an der Carbamat-Gruppe degradieren.

### 7.2 Vernetzung von O-(Methacryloxyethyl)-N-(triethoxysilylpropyl)urethan durch hydrolytische Kondensation der Silyl-Gruppen

In einem 50 ml Rundkolben wurden 15,17 g *O*-(Methacryloxyethyl)-*N-*(triethoxysilylpropyl)urethan in 25 ml Ethylacetat gelöst. Anschließend wurden 1,8 ml 0,5 M Salzsäure (Lösung aus 192 ml Wasser und 8 ml konz. Salzsäure) zugegeben und die weißlich trübe Lösung für 42 Stunden unter Lichtausschluss gerührt. Danach wurde 3 Mal mit je 23 ml destilliertem Wasser gewaschen und nach jedem Waschschritt der pH-Wert kontrolliert. Nach Trocknen der Mischung mit Hilfe von hydrophobem Filterpapier wurde das Lösungsmittel destillativ entfernt und eine viskose, leicht gelbliche Flüssigkeit erhalten.

¹H-NMR ([D₆]Aceton, 23°C, 400,1 MHz, [ppm]): δ = 0,66 (m, 2 H, NHCH₂C*H₂*CH₂Si), 1,66 (m, 2 H, NHCH₂C*H₂*CH₂Si), 1,91 (s, 3 H, H₂C=CC*H₃*C(O)O), 3,14 (m, 2 H, NHCH₂C*H₂*CH₂Si), 4,30 (m, 4 H, C(O)OC*H₂*C*H₂*OC(O)), 5,62 (m, 1 H, O(O)CCCH₃=C*H₂ cis*), 6,08 (m, 1 H, O(O)CCCH₃=*CH₂ trans*). -²⁹Si-NMR ([D₆]Aceton, 23°C, 79,50 MHz, [ppm]): δ = -66,52 - -63,88 (m, T³, NHCH₂CH₂CH₂*Si*(OSi)₃), -60,64 - -59,49 (m, T², NHCH₂CH₂CH₂*Si*(OSi)₂(OEt)), -57,99--57,41 (m, T², NHCH₂CH₂CH₂*Si*(OSi)₂(OH)), -56,11 - -48,77 (m, T¹,

NHCH₂CH₂CH₂*Si*(OSi)(OEt)₂, NHCH₂CH₂CH₂*Si*(OSi)(OEt)(OH), NHCH₂CH₂CH₂*Si*(OSi)(OH)₂). FT-IR (ATR, 23°C, [cm⁻¹]): *υ̃* = 3338 (m, v(N-H)), 1698 (s, v(C=O)), 1636 (m, v(C=C)), 1530 (s, v(CHN)), 1450 (m, δₐₛ(C-H)), 1246 (s, vₐₛ(C-O-C)), 1045 (s, v(Si-O-Si)).

µ-Raman (23°C, [cm⁻¹]): *υ̃* = 539 (m, v(C=O)), 1295 (m, v(C-O-C)), 1407 (s, δ(=CH₂)), 1444 (s, δ(CH₂)), 1639 (s, v(C=C)), 1718 (s, v(C=O)), 3363 (w, v(N-H)).

Ausbeute: 12,79 g

### 7.3 Polymerisation des Kondensats durch UV-Bestrahlung

Das gemäß 7.2 hergestellte Harz (Polykondensat) wurde mit 2 Gew.-% Irgacure^{®} 369 versetzt, in eine Form gefüllt und mit Hilfe von UV-Belichtung organisch polymerisiert (ausgehärtet). Das IR-Spektrum vor und nach der Polymerisation ist in **Figur 14** dargestellt. Man erkennt eine deutliche Abnahme der C=C-Bande bei 1640 cm⁻¹ im Verhältnis zur C=O-Bande bei 1718 cm⁻¹ als Folge der organischen Vernetzung.

### 7.4 Herstellung von Formkörpern mittels 2-Photonen-Absoprtion

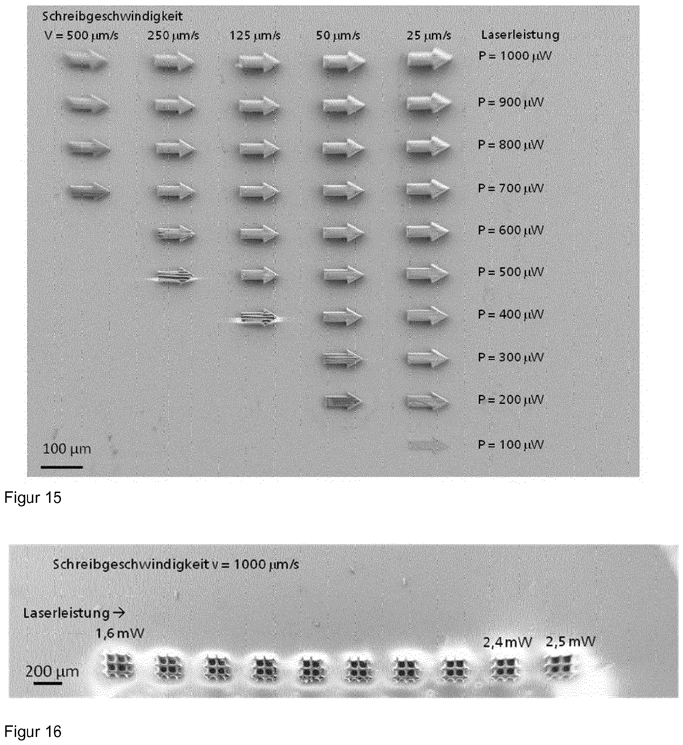

Das gemäß 7.2 hergestellte Harz (Polykondensat) wurde mittels Zwei-Photonen-Absorption (TPA) organisch polymerisiert und wie oben im allgemeinen Beispiel "Mehrphotonen-polymerisation" angegeben strukturiert. Es wurden unterschiedliche Schreibgeschwindigkeiten und Laserleistungen verwendet. **Figur 15** zeigt eine Reihe von Ergebnissen im Vergleich. Besonders feine Strukturierungen (Ausbildung von Kanälen, Vorstudie für die Erzeugung von Scaffolds) sind in **Figur 16** gezeigt. Hier wurde die Laserleistung bei einer gleichbleibenden Schreibgeschwindigkeit von 1000 µm/s schrittweise von 1,6mW auf 2,5mW erhöht.

### 7.5 Scaffold-Herstellung

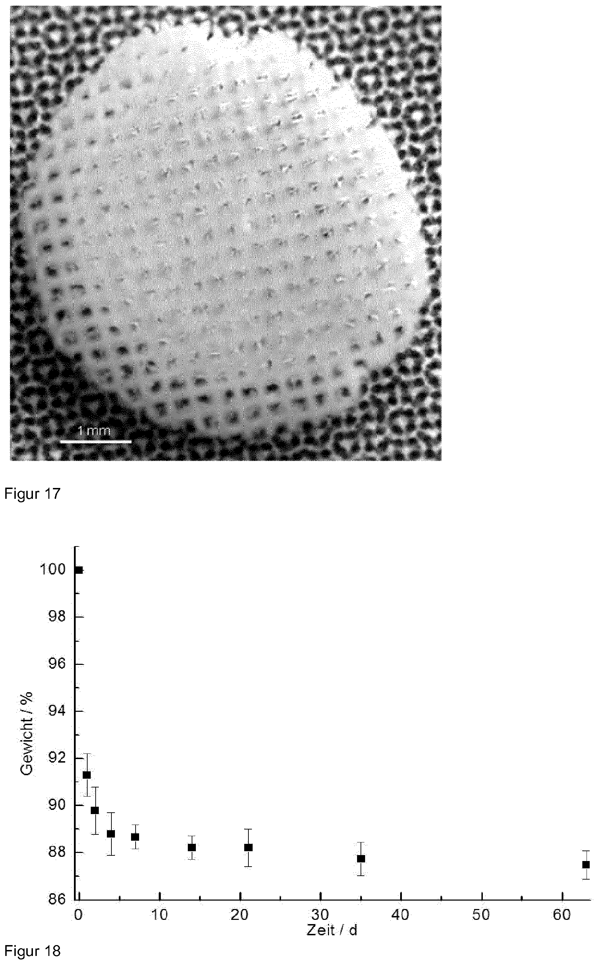

Das gemäß 7.2 hergestellte Harz (Polykondensat) wurde mittels Zwei-Photonen-Absorption (TPA) organisch polymerisiert und wie oben im allgemeinen Beispiel "Mehrphotonen-polymerisation" angegeben unter den nachfolgend angegebenen Bedingungen und mit den angegebenen Abmessungen strukturiert. Das Scaffold ist in **Figur 17** gezeigt.
- Design:
   o Durchmesser 5,5 mm, Höhe ca. 1,2 mm
   o kubische Poren (200 Mikrometer Seitenlänge)
- Prozessierung
   1. Zweiphotonenpolymerisation mit Standardbelichtungsaufbau (siehe Figur 1, die auch detailliert in WO 03/037606 A1 erläutert ist)
   2. Entwicklung (3 h in Isopropanol/MBIK (1:1))
   3. UV Nachbelichtung mit dem Maskaligner (20 mW/cm² für 3 min)

### 7.6 Degradationsstudie

Das gemäß 7.2 hergestellte Harz (Polykondensat) wurde wie im allgemeinen Beispiel erläutert ausgehärtet und danach in PBS-Lösung eingelegt. Das Ergebnis ist graphisch in **Figur 18** dargestellt.

### 7.7 Biokompatibilitätsstudie

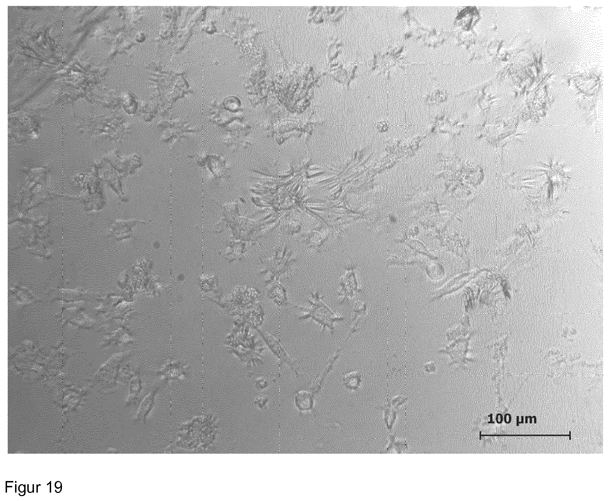

Das gemäß 7.2 hergestellte Harz wurde mit UV-Licht bestrahlt. Nach dem Aushärten wurde ein Biokompatibilitätsversuch mit Mausfibroblasten L929 durchgeführt, die auf das ausgehärtete Material (Bulkmaterial) aufgebracht wurden. Nach sieben Tagen war das Material mit Zellen besiedelt. Diese hatten sich in alle Richtungen ausgestreckt ("Spreading"), wie in **Figur 19** zu erkennen, was ein Zeichen dafür ist, dass es ihnen gut geht.

## Patentansprüche

1. Verfahren zum Herstellen einer Verbindung mit der Formel (A)
R'-CHR"-O-C(O)-CR‴=CH₂ (A)
worin der Rest R‴ H oder CH₃ bedeutet und worin R'CHR"- abgeleitet ist von einer Verbindung R'-CHR"OH, ausgewählt unter C-6-Pyranosen, Furanosen und davon abgeleiteten reinen oder gemischten Di- oder Polysacchariden, in denen pro Zuckermolekül zweimal jeweils zwei Hydroxygruppen durch eine Isopropylidengruppe geschützt sind, Milchsäure, Polylactaten und Polymeren von γ-Butyro-, Valerio- und Caprolacton,
**dadurch gekennzeichnet, dass** eine Verbindung
R'-CHR"OH
mit einer Verbindung
R^{IV}O-C(O)-CR‴=CH₂
worin R^{IV} ein mit einer oder mehreren Gruppen O-C(O)-CR‴=CH₂ substituierter Alkylrest ist, unter Bedingungen umgesetzt wird, die das Reaktionsgleichgewicht zum Produkt hin verschieben.

2. Verfahren nach Anspruch 1, worin der Alkylrest R^{IV} 1 bis 20, bevorzugt 1 bis 10 Kohlenstoffatome aufweist.

3. Verfahren nach Anspruch 1, worin der Alkylrest R^{IV} CH₂-CH₂-O-C(O)-CR‴=CH₂ ist, worin R‴ die in Formel (A) von Anspruch 1 angegebene Bedeutung besitzt.

4. Verfahren nach einem der voranstehenden Ansprüche, worin das Reaktionsgleichgewicht mit Hilfe eines Überschusses an der Verbindung R^{IV}O-C(O)-CR^{III}=CH₂ in der Reaktionsmischung und/oder durch einen Katalysator, insbesondere ein Alkoxytitanat, zum Produkt hin verschoben wird.

## Claims

1. A process for preparing a compound having the formula (A)
R'-CHR"-O-C(O)-CR‴=CH₂ (A)
wherein the radical R'" is H or CH₃ and wherein R'CHR" is derived from a compound R'-CHR"OH selected from C-6 pyranoses, furanoses and pure or mixed di- or polysaccharides derived therefrom in which two times two hydroxy groups per sugar molecule are protected by an isopropylidene group, lactic acid, polylactates and polymers of γ-butyro-, valerio- and caprolactone,
**characterised in that** a compound
R'-CHR "OH
is reacted with a compound
R^{IV}O-C(O)-CR‴=CH₂
wherein R^{IV} is an alkyl radical substituted with one or more groups OC(O)-CR‴=CH₂,
under conditions which shift the reaction equilibrium towards the product.

2. The process according to claim 1, wherein the alkyl radical R^{IV} has 1 to 20, preferably 1 to 10 carbon atoms.

3. The process according to claim 1, wherein the alkyl radical R^{IV} is CH₂-CH₂-O-C(O)-CR‴=CH₂, wherein R‴ has the meaning given in formula (A) of claim 1.

4. The process according to any one of the preceding claims, wherein the reaction equilibrium is shifted towards the product with the aid of an excess of the compound R^{IV}O-C(O)-CR^{III}=CH₂ in the reaction mixture and/or by a catalyst, in particular an alkoxy titanate.

## Revendications

1. Procédé de fabrication d'un composé de formule (A)
R'-CHR"O-C(O)-CR‴=CH₂ (A)
dans lequel le radical R signifie R‴ H ou CH₃ et où R'CHR"-est dérivé d'un composé R'-CHR"OH, choisi parmi les pyranoses à 6C, les furanoses et les disaccharides ou polysaccharides purs ou mélangés dérivés de ceux-ci, dans lesquels deux groupes hydroxy sont respectivement protégés deux fois par un groupe d'isopropylidène pour chaque molécule de sucre, les acides lactiques, les polylactates et les polymères de γ-butyrolactone, de valerolactone et de caprolactone,
**caractérisé en ce que**
un composé
R'-CHR"OH
avec un composé
R^{IV}O-C(O)-CR‴=CH₂
où R^{IV} est un radical alkyle substitué par un ou plusieurs groupes O-C(O)-CR‴=CH₂,
est transformé dans des conditions qui reportent l'équilibre de réaction vers le produit.

2. Procédé selon la revendication 1, dans lequel le radical alkyle R^{IV} représente 1 à 20, de préférence 1 à 10, atomes de carbone.

3. Procédé selon la revendication 1, dans lequel le radical alkyle R^{IV} est CH₂-CH₂-O-C(O)-CR‴=CH₂, où R‴ a la signification mentionnée dans la formule (A) de la revendication 1.

4. Procédé selon une des revendications précédentes, dans lequel l'équilibre de réaction est reporté vers le produit à l'aide d'un excédent de composé R^{IV} 0-C(O)-CR^{III}=CH² dans le mélange de réaction et/ou par un catalyseur, en particulier un alkoxy titanate.
